(19) **Europäisches Patentamt**

**European Patent Office**

**Office européen des brevets**

(11) Veröffentlichungsnummer: **0 396 977 B1**

(12) **EUROPÄISCHE PATENTSCHRIFT**

(45) Veröffentlichungstag der Patentschrift: **15.06.94**

(51) Int. Cl.⁵: **C07C 265/14**, C07C 263/04, C07C 263/20

(21) Anmeldenummer: **90108013.5**

(22) Anmeldetag: **27.04.90**

(54) **Verfahren zur Herstellung von Polyisocyanaten.**

(30) Priorität: **10.05.89 DE 3915181**

(43) Veröffentlichungstag der Anmeldung:
**14.11.90 Patentblatt 90/46**

(45) Bekanntmachung des Hinweises auf die Patenterteilung:
**15.06.94 Patentblatt 94/24**

(84) Benannte Vertragsstaaten:
**BE DE ES FR GB IT NL**

(56) Entgegenhaltungen:
**EP-A- 0 061 013**
**EP-A- 0 092 738**
**EP-A- 0 175 117**
**US-A- 3 919 280**

(73) Patentinhaber: **BAYER AG**

**D-51368 Leverkusen(DE)**

(72) Erfinder: **Hammen, Günter, Dr.**
**Bunzlauer Weg 13**
**D-4049 Romerskirchen 1(DE)**
Erfinder: **Knöfel, Hartmut, Dr.**
**Dülmener Weg 21**
**D-5068 Odenthal-Erberich(DE)**
Erfinder: **Friederichs, Wolfgang, Dr.**
**Geilenkircher Strasse 45**
**D-5000 Köln 41(DE)**

**Beschreibung**

Die Erfindung betrifft ein Verfahren zur Herstellung von organischen Polyisocyanaten durch thermische Spaltung der ihnen zugrundeliegenden Carbaminsäureester.

Es ist seit langem bekannt, daß N-substituierte Urethane thermisch in der Gasphase oder in flüssiger Phase in Isocyanate und Alkohol spaltbar sind [A. W. Hofmann, Ber. Dtsch. Chem. Ges. 3, 653 (1870); H. Schiff, Ber. Dtsch. Chem. Ges. 3, 649 (1870)].

In der US-PS 2 409 712 ist ein Verfahren beschrieben, bei dem die bei der Spaltung von Carbaminsäureestern in Substanz entstehenden Produkte durch Einleiten in ein Cyclohexan/Wasser-Gemisch an der Rekombination gehindert werden sollen. Dieses Verfahren liefert infolge partieller Hydrolyse der entstehenden Isocyanate an der Phasengrenze nur mäßige Isocyanatausbeuten.

In Anwesenheit inerter hochsiedender Lösungsmittel verlaufen die Verfahren z. B. gemäß US-PS 3 962 302 und US-PS 3 919 278. Dabei werden die beiden Spaltprodukte, also Alkohol und Isocyanat, gemeinsam kontinuierlich aus dem Spaltmedium abdestilliert und durch fraktionierte Kondensation getrennt. Nachteilig bei diesem Verfahren sind der hohe technische Aufwand zur Trennung von Alkohol- und Isocyanatdampf und die nur mäßige Ausbeute. Leichtflüchtige Isocyanate lassen sich infolge der starken Verdünnung und des daraus resultierenden geringen Partialdampfdrucks nur schwer aus dem Spaltmedium abdestillieren.

Bei dem Verfahren gemäß US 3 919 279, DE-OS 2 635 490 oder DE-OS 2 942 543 werden zur Erhöhung der Raum/Zeit-Ausbeute homogene bzw. heterogene Katalysatoren mitverwendet. Gemäß EP-A 0 061 013 sollen durch Zugabe stabilisierender Zusätze Isocyanatfolgereaktionen unterdrückt werden. Die Schwierigkeiten, die sich bei der erforderlichen Destillation der Isocyanate ergeben, lassen sich jedoch durch diese Zusätze nicht verringern.

Gemäß EP-A 0 092 738 lassen sich Isocyanatfolgereaktionen durch kurze Verweilzeiten unterdrücken. Dazu wird der geschmolzene Carbaminsäureester in Gegenwart geringer Mengen eines Hilfslösungsmittels an der Innenwand eines Rohrreaktors entlanggeführt, die entstehenden hochsiedenden Nebenprodukte sowie das Hilfslösungsmittel als Sumpfprodukt ausgeschleust und die aus Isocyanat und Alkohol bestehenden Spaltgase über Kopf abgenommen und durch fraktionierte Kondensation getrennt. Da bei diesem Verfahren nur mit geringen Mengen an Hilfslösungsmitteln gearbeitet wird, die Carbaminsäureester also nahezu unverdünnt einer Thermolyse unterworfen werden, läßt sich die Bildung hochviskoser polymerer Nebenprodukte nicht vermeiden. Darüber hinaus ist das über Kopf abgenommene Isocyanat infolge partieller Rekombination mit dem ebenfalls über Kopf abgenommenen Alkohol stets durch Carbaminsäureester verunreinigt.

Es war die der Erfindung zugrundeliegende Aufgabe, ein neues Verfahren zur Herstellung von organischen Polyisocyanaten durch thermische Spaltung der den Polyisocyanaten entsprechenden Carbaminsäureester zur Verfügung zu stellen, bei welchem die entstehenden Polyisocyanate unter Vermeidung der Bildung von Nebenprodukten und unter Verhinderung einer Rekombination mit dem gebildeten Alkohol hergestellt werden können.

Diese Aufgabe konnte mit dem nachfolgend näher erläuterten erfindungsgemäßen Verfahren gelöst werden.

Gegenstand der Erfindung ist ein Verfahren zur Herstellung von Polyisocyanaten durch thermische Spaltung der den Polyisocyanaten entsprechenden N-substituierten Carbaminsäureestern in Lösung in Gegenwart von bis zu 75 Gew.-% eines Lösungsmittels oder Lösungsmittelgemisches bei oberhalb 150 °C liegenden Temperaturen, dadurch gekennzeichnet, daß man

A) den Carbaminsäureester oder das Gemisch von Carbaminsäureestern der Formel

$$R^1(NHCOOR^2)_n$$

in welcher

R$^1$ für einen gegebenenfalls inerte Substituenten aufweisenden aliphatischen Kohlenwasserstoffrest mit insgesamt 4 bis 18 Kohlenstoffatomen, einen gegebenenfalls inerte Substituenten aufweisenden cycloaliphatischen Kohlenwasserstoffrest mit insgesamt 6 bis 25 Kohlenstoffatomen, einen gegebenenfalls inerte Substituenten aufweisenden araliphatischen Kohlenwasserstoffrest mit insgesamt 7 bis 25 Kohlenstoffatomen oder einen gegebenenfalls inerte Substituenten aufweisenden aromatischen Kohlenwasserstoffrest mit insgesamt 6 bis 30 Kohlenstoffatomen steht,

R$^2$ für einen aliphatischen Kohlenwasserstoffrest mit 1 bis 18 Kohlenstoffatomen, einen cycloaliphatischen Kohlenwasserstoffrest mit 5 bis 15 Kohlenstoffatomen, einen araliphatischen Kohlenwasserstoffrest mit 7 bis 10 Kohlenstoffatomen oder einen aromatischen Kohlenwasserstoffrest mit 6 bis 10 Kohlenstoffatomen steht, und

n für eine ganze Zahl von 2 bis 5 steht,
mit der Maßgabe, daß die dem Rest R$^2$ entspre-

chenden Alkohole $R^2$-OH bei Normaldruck einen mindestens um 10 ° C unterhalb des Siedepunktes des eingesetzten Lösungsmittels und des dem Rest $R^1$ entsprechenden Polyisocyanats $R^1(NCO)_n$ liegenden Siedepunkt aufweisen, als mindestens 25 gew.-%ige Lösung in einem polaren Lösungsmittel oder Lösungsmittelgemisch löst, das

a) eine Dielektrizitätskonstante bei 25 ° C oberhalb 20 aufweist,

b) die Carbaminsäureester zu lösen vermag,

c) bei der Spalttemperatur stabil und gegenüber den Carbaminsäureestern und den gebildeten Isocyanaten inert ist,

d) einen Siedepunkt mindestens 10 ° C oberhalb des Siedepunktes des dem zu spaltenden Carbaminsäureester Zugrundeliegenden Alkohols aufweist,

e) unter den Spaltbedingungen unzersetzt destillierbar ist,

f) mit dem gemäß D) eingesetzten Extraktionsmittel mindestens eine Mischungslücke aufweist, und

B) die thermische Spaltung in einem Rohrreaktor so durchführt, daß Gemische aus Polyisocyanaten, Isocyanatourethanen und unumgesetzten Carbaminsäureestern erhalten werden, deren Isocyanatgehalt mehr als 50 % der Theorie entspricht, wobei die gemäß A) hergestellten Lösungen an der Innenwand des Rohrreaktors entlang geführt werden,

C) die unter den Reaktionsbedingungen anfallenden Gase über Kopf abnimmt und durch fraktionierte Kondensation in eine vorwiegend aus Alkohol bestehende Fraktion I und eine vorwiegend aus Polyisocyanat, Isocyanatourethan, Carbaminsäureester und dem gemäß A) eingesetzten Lösungsmittel bestehende Fraktion II auftrennt,

D) das in Fraktion II enthaltene Polyisocyanat durch Extraktion mit einem Extraktionsmittel mit einem bei 1013 mbar bei 30 ° C bis 200 ° C liegenden Siedepunkt bzw. -bereich, welches mit der Fraktion II nicht unbegrenzt mischbar ist und für das Polyisocyanat ein Lösungsmittel darstellt und welches aus der Gruppe der aliphatischen, cycloaliphatischen oder araliphatischen Kohlenwasserstoffe, aliphatischen Ether oder beliebiger Gemische derartiger Lösungsmittel ausgewählt ist, aus Fraktion II entfernt und gegebenenfalls anschließend die dabei anfallende Lösung des Polyisocyanates in dem Extraktionsmittel destillativ aufarbeitet, und

E) den nicht extrahierten Teil des Reaktionsgemisches zurückführt, vorzugsweise in die Spaltungsreaktion gemäß B).

In einer bevorzugten Verfahrensvariante wird als Lösungsmittel Sulfolan oder 3-Methyl-sulfolan verwendet.

Besonders bevorzugt als Extraktionsmittel sind Isooctan, Cyclohexan, Toluol und/oder tert.-Butylmethylether.

Vorzugsweise werden beim erfindungsgemäßen Verfahren solche Carbaminsäureester der genannten Formel eingesetzt, für welche

$R^1$    für einen aliphatischen Kohlenwasserstoffrest mit insgesamt 4 bis 12, insbesondere 6 bis 10 Kohlenstoffatomen, einen cycloaliphatischen Kohlenwasserstoffrest mit 6 bis 15 Kohlenstoffatomen, einen Xylylenrest oder einen aromatischen, gegebenenfalls Methylsubstituenten und/oder Methylenbrücken aufweisenden Kohlenwasserstoffrest mit insgesamt 7 bis 30 Kohlenstoffatomen steht,

$R^2$    für einen aliphatischen Kohlenwasserstoffrest mit 1 bis 6, insbesondere 1 bis 4 Kohlenstoffatomen, einen Cyclohexylrest oder einen Phenylrest steht, und

n      für eine ganze Zahl von 2 bis 5 steht.

Typische Beispiele für geeignete Carbaminsäureester sind

1-(n-Butoxicarbonylamino)-3,3,5-trimethyl-5-(n-butoxicarbonylamino-methyl)-cyclohexan
1-Methyl-2,4-bis-(ethoxicarbonylamino)-benzol
1-Methyl-2,6-bis-(ethoxicarbonylamino)-benzol
1,10-Bis-(methoxicarbonylamino)-decan
1,12-Bis-(butoxicarbonylamino)-dodecan
1,12-Bis-(methoxicarbonylamino)-dodecan
1,12-Bis-(phenoxicarbonylamino)-dodecan
1,18-Bis-(2-butoxiethylcarbonylamino)-octadecan
1,18-Bis-(benzyloxicarbonylamino)-octadecan
1,3-Bis-(ethoxicarbonylamino-methyl)-benzol
1,3-Bis-(methoxicarbonylamino)-benzol
1,3-Bis-[(methoxicarbonylamino)-methyl]-benzol
1,3,6-Tris-(methoxicarbonylamino)-hexan
1,3,6-Tris-(phenoxicarbonylamino)-hexan
1,4'-Bis-[(3-isopropyl-5-methylphenoxi)-carbonylamino]-butan
1,4-Bis-(ethoxicarbonylamino)-butan
1,4-Bis-(ethoxicarbonylamino)-cyclohexan
1,5-Bis-(butoxicarbonylamino)-naphthalin
1,6-Bis-(ethoxicarbonylamino)-hexan
1,6-Bis-(methoxicarbonylamino)-hexan
1,6-Bis-(methoximethylcarbonylamino)-hexan
1,8-Bis-(ethoxicarbonylamino)-octan
1,8-Bis-(phenoxicarbonylamino)-4-(phenoxicarbonylaminmethyl)-octan
2,2'-Bis-(4-propoxicarbonylamino-phenyl)-propan
2,2'-Bis-(methoxicarbonylamino)-diethylether
2,4'-Bis-(ethoxicarbonylamino)-diphenylmethan
2,4-Bis-(methoxicarbonylamino)-cyclohexan
4,4'-Bis-(ethoxicarbonylamino)-dicyclohexylmethan
2,4'-Bis-(ethoxicarbonylamino)-diphenylmethan
4,4'-Bis-(methoxicarbonylamino)-2,2-dicyclohexylpropan

4,4'-Bis-(methoxicarbonylamino)-biphenyl
4,4'-Bis-(n-butoxicarbonylamino)-2,2-dicyclohexylpropan
4,4'-Bis-(phenoxicarbonylamino)-dicyclohexylmethan
4,4'-Bis-(phenoxicarbonylamino)-diphenylmethan
Als Lösungsmittel, das heißt als Reaktionsmedium für die Durchführung des erfindungsgemäßen Verfahrens kommen polare Lösungsmittel in Betracht, die unter Spaltbedingungen einen mindestens 10°C oberhalb des Siedepunktes des dem Carbaminsäureester zugrundeliegenden Alkohols liegenden Siedepunktes aufweisen und die folgenden Bedingungen erfüllen:

a) Sie müssen unter den an späterer Stelle beschriebenen Extraktionsbedingungen sowohl die als Ausgangsmaterialien eingesetzten Carbaminsäureester als auch die als Reaktionsprodukte anfallenden Isocyanate lösen,

b) Sie müssen unter den Spaltbedingungen thermisch weitgehend stabil sein,

c) Sie müssen gegenüber den eingesetzten Carbaminsäureestern und den entstehenden Isocyanaten chemisch inert sein,

d) Sie müssen mit den gemäß Stufe D) des erfindungsgemäßen Verfahrens eingesetzten Extraktionsmitteln mindestens eine Mischungslükke aufweisen.

Diesen Kriterien entsprechende, für das erfindungsgemäße Verfahren als Reaktionsmedium geeignete Lösungsmittel sind beispielsweise aliphatische Sulfone wie Diethylsulfon, Dipropylsulfon, Dibutylsulfon, Ethyl-propylsulfon; cyclische Sulfone wie Sulfolan, 2-Methylsulfolan, 3-Methylsulfolan, 2,4-Dimethylsulfolan; araliphatische Sulfone wie Methylphenylsulfon, Ethylphenylsulfon; aromatische Sulfone wie Diphenylsulfon, (4-Methylphenyl)-phenylsulfon; aromatische Nitroverbindungen wie Nitrobenzol, 2-Nitrotoluol, 3-Nitrotoluol), 4-Chlornitrobenzol; sowie Gemische derartiger Verbindungen. Bevorzugt werden Sulfolan, 3-Methylsulfolan und Nitrobenzol, besonders bevorzugt Sulfolan als Lösungsmittel eingesetzt.

Als Extraktionsmittel eignen sich insbesondere aliphatische oder cycloaliphatische Kohlenwasserstoffe oder aliphatische Ether, die bei 1013 mbar einen Siedepunkt zwischen 30°C und 200°C, vorzugsweise 30°C bis 150°C, aufweisen. Geeignet sind beispielsweise n-Hexan, i-Octan, den genannten Definitionen entsprechende Benzinfraktionen, Cyclohexan oder Methylcyclohexan bzw. aliphatische Ether mit mindestens 4 Kohlenstoffatomen, vorzugsweise 4 bis 12 Kohlenstoffatomen, beispielsweise Diethylether, isomere Butylether, tert.-Butyl-methyl-ether oder n-Heptyl-methyl-ether. Aromatische Kohlenwasserstoffe wie Benzol, Toluol oder Xylol sind zwar ebenfalls geeignet, jedoch weniger bevorzugt. Die beispielhaft genannten aliphatischen oder cycloaliphatischen Kohlenwasserstoffe sind die besonders bevorzugten Extraktionsmittel. Beliebige Gemische der beispielhaft genannten Extraktionsmittel können selbstverständlich ebenfalls verwendet werden.

Die Durchführung des erfindungsgemäßen Verfahrens erfolgt im allgemeinen, indem man den zu spaltenden Carbaminsäureester gegebenenfalls unter Zusatz von bis zu 10 Mol-%, vorzugsweise bis zu 1 Mol-% eines Katalysators, als mindestens 25 gew.-%ige, vorzugsweise mindestens 40 gew.-%ige Lösung in einem als Reaktionsmedium dienenden Lösungsmittel oder Lösungsmittelgemisch der obengenannten Art bei einem Druck von 0,001 bis 5 bar in einer dünnen Schicht an der Innenwand eines auf Temperaturen von 150°C bis 400°C beheizten Rohres entlangführt. Die Verweilzeit des Carbaminsäureesters im Reaktionsrohr wird dabei zur Unterdrückung von Nebenreaktionen sehr kurz gehalten, und die Spaltprodukte werden gemeinsam mit mindestens einem Teil des eingesetzten Lösungsmittels als gasförmige Produkte rasch aus der Reaktionszone entfernt und an zwei hintereinander angeordneten, geeignet temperierten Dephlegmatoren selektiv kondensiert, so daß eine hauptsächlich aus Alkohol bestehende Fraktion I und eine hauptsächlich aus Isocyanat und Lösungsmittel bestehende Fraktion II, die gegebenenfalls die durch unvollständige Spaltung oder Rekombination entstehenden Isocyanatourethane und/oder Carbaminsäureester enthält, anfallen.

Die für das erfindungsgemäße Verfahren geeigneten Reaktionsrohre können sehr unterschiedlich konstruiert sein. Sie müssen lediglich so betrieben werden können, daß die in das Rohr eingespeisten Carbaminsäureester sich in einer dünnen Schicht auf der beheizten Rohrinnenwand verteilen können und die gasförmigen und/oder flüssigen Spaltprodukte aus dem Reaktionsrohr entnommen werden können.

Die Verteilung der eingespeisten Carbaminsäureester auf der Rohrinnenwand kann in senkrecht stehenden Rohrreaktoren ohne Hinzuziehung besonderer Vorrichtungen geschehen, sofern der zu spaltende Carbaminsäureester bzw. dessen Lösung in einem Lösungsmittel über eine geeignete Vorrichtung, z B. eine Düse, gleichmäßig auf die Rohrwandung aufgetragen wird. Sie kann aber auch unter Zuhilfenahme eines mechanischen Rührwerks oder ähnlicher Einrichtungen bewerkstelligt werden. In nicht senkrecht stehenden Rohrreaktoren ist die Verwendung eines Rührwerks oder einer anderen geeigneten Vorrichtung in den meisten Fällen notwendig.

Die zur Erzeugung bzw. Verbesserung des Flüssigkeitsfilms eingesetzten Rührwerke können vorteilhaft auch zum Transport des an der Rohrwandung befindlichen Materials benutzt werden, sei es, daß sie die abwärts gerichtete Fließbewegung des Flüssigkeitsfilms hemmen, sei es, daß sie in schräg oder waagerecht liegenden Reaktoren die eingespeisten, in Lösungsmitteln gelösten Carbaminsäureester und/oder deren Zersetzungsprodukte über die Spaltzone hinweg zum Rohrende befördern.

Die hochsiedenden Nebenprodukte werden, gegebenenfalls zusammen mit einem Teil des als Spaltungsmedium eingesetzten Lösungsmitels oder Lösungsmittelgemischs, als Sumpfprodukt ausgeschleust.

Geeignete Spaltreaktoren sind z. B. als Fallfilmverdampfer arbeitende Glas-, Quarz- oder Metallrohre, mit schneckenartigen Rührwerken ausgestattete, gegebenenfalls zum Ende sich verjüngende Rohrreaktoren oder auch herkömmliche Dünnschichtverdampfer in diversen Ausführungsformen. Von diesen haben sich mit mechanischen Rührwerken ausgestattete Dünnschichtverdampfer als besonders günstig erwiesen.

Das erfindungsgemäße Verfahren verläuft dann ordnungsgemäß, wenn im herablaufenden Flüssigkeitsfilm eine kontinuierliche Verarmung an eingespeisten Carbaminsäureestern erfolgt und am Ende der Spaltzone kein oder nur sehr wenig Ausgangsmaterial ankommt. Das erfindungsgemäße Verfahren zur thermischen Spaltung von Carbaminsäureestern kann natürlich auch derart durchgeführt werden, daß man die eingebrachten Carbaminsäureester nur sehr unvollständig spaltet, indem man als Fraktion II ein Produkt, das überwiegend oder zumindest teilweise Isocyanatourethane und/oder Carbaminsäureester enthält, abnimmt.

Die bei der thermischen Spaltung von Carbaminsäureestern entstehende Fraktion II, die vorwiegend die herzustellenden Polyisocyanate, daneben aber auch Reste von nicht oder nur teilweise gespaltenen Carbaminsäureestern enthalt, wird in einem zweiten Verfahrensschritt einer Extraktion unterworfen. Falls erwünscht, kann die Fraktion II selbstverständlich zuvor mit weiteren Lösungsmitteln der oben genannten Art verdünnt werden.

Zur Durchführung des erfindungsgemäßen Extraktionsschritts wird die isocyanathaltige Fraktion II mit einem bei Raumtemperatur flüssigen Extraktionsmittel der oben beispielhaft genannten Art intensiv durchmischt, wobei, bezogen auf die zu extrahierende Fraktion II, das Extraktionsmittel in einer 0,1 bis 25fachen, vorzugsweise 0,5 bis 5fachen Gewichtsmenge zum Einsatz gelangt.

Die Durchmischung der Fraktion II mit dem Extraktionsmittel erfolgt im allgemeinen innerhalb des Temperaturbereichs von -20°C bis 150°C, vorzugsweise bei 10°C bis 100°C.

Im allgemeinen entsteht hierbei ein zweiphasiges, aus zwei flüssigen Phasen bestehendes Gemisch, welches nach Phasenabscheidung in eine spezifisch leichtere und eine spezifisch schwerere Phase getrennt werden kann. Die Bildung eines zweiphasigen Systems kann in besonderen Fällen jedoch auch durch eine Abkühlung des Gemisches der Fraktion II mit dem Extraktionsmitel begünstigt werden. So ist es beispielsweise möglich, die Durchmischung bei ca. 70°C bis 100°C vorzunehmen und anschließend das Gemisch auf eine tiefere Temperatur beispielsweise im Temperaturbereich von 10°C bis 40°C abzukühlen. In dem sich bildenden zweiphasigen System bildet die spezifisch leichtere Phase im allgemeinen die Hauptphase und die spezifisch schwerere Phase die Nebenphase, wobei das Volumenverhältnis weitgehend von der Menge des eingesetzten Extraktionsmittels abhängt. Die Phasentrennung kann bei der Durchführung des erfindungsgemäßen Verfahrens nach an sich bekannten Methoden, beispielsweise durch Ablassen der schwereren Phase, Dekantieren, Abhebern oder andere geeignete Methoden der Phasentrennung vorgenommen werden. Ein Teil des in reiner Form zu gewinnenden Polyisocyanats liegt dann in der spezifisch leichteren Hauptphase vor. Weitere Bestandteile der spezifisch leichteren Phase sind ein Teil des als Spaltmedium verwendeten Lösungsmittels sowie die Hauptmenge des eingesetzten Extraktionsmittels. Die spezifisch schwerere Phase besteht hauptsächlich aus dem als Spaltmedium verwendeten Lösungsmittel, den nicht oder nur teilweise umgesetzten Carbaminsäureestern sowie dem nicht in die spezifisch leichtere Phase übergegangenen Teil des zu gewinnenden Polyisocyanats. Um auch diesen Teil an Polyisocyanat in reiner Form zu gewinnen, kann die spezifisch schwerere Phase einer oder mehrerer erneuter Extraktionen in der beschriebenen Art und Weise unterworfen werden.

Gemäß einer besonderen Ausführungsform des erfindungsgemäßen Verfahrens erfolgt die Durchmischung der Fraktion II mit dem Extraktionsmittel und die anschließende Phasentrennung, d. h. die Extraktion der Fraktion II, kontinuierlich unter Verwendung der üblichen, kontinuierlich arbeitenden Gegenstromextraktionsapparaturen.

Als Ergebnis der Extraktion resultieren eine oder mehrere, gegebenenfalls zu vereinigende, spezifisch leichtere Extraktphasen, die das zu gewinnende Polyisocyanat enthalten und eine, in der Regel homogene, zweite Phase, die vorwiegend nicht oder nur unvollständig umgesetzte Carbaminsäureester enthält. Diese zweite, spezifisch schwerere Phase kann in die Spaltungsreaktion zurückgeführt werden.

Zur Gewinnung der Polyisocyanate in reiner Form werden die genannten spezifisch leichteren Extraktphasen destillativ aufgearbeitet, wobei im allgemeinen das Extraktionsmittel die zunächst destillativ zu entfernende Fraktion darstellt. Auch die Trennung der Polyisocyanate von Restmengen des Lösungsmittels kann destillativ erfolgen, wobei das Polyisocyanat oder das als Spaltmedium eingesetzte Lösungsmittel den Destillationsrückstand bildet. Vorzugsweise wird man solche Lösungsmittel der oben beispielhaft genannten Art verwenden, die sich bezüglich ihres Siedeverhaltens ausreichend deutlich vom zu gewinnenden Polyisocyanat unterscheiden, so daß eine einwandfreie Auftrennung möglich ist. Im allgemeinen stellt das zu gewinnende Polyisocyanat bei der destillativen Aufarbeitung der spezifisch leichteren Extraktphasen den Destillationsrückstand dar. Auch die destillative Aufarbeitung kann kontinuierlich unter Verwendung der bekannten Destillationsapparaturen erfolgen. Die im allgemeinen als Destillationsrückstand anfallenden Polyisocyanate können gegebenenfalls einer weiteren Feindestillation unterzogen werden. Jedoch auch ohne derartige Feindestillation weisen die als Destillationsrückstände anfallenden Polyisocyanate bereits einen Reinheitsgrad von zum Teil über 90 Gew.-% auf.

Es ist ein besonderer Vorteil des erfindungsgemäßen Verfahrens, daß die bei der Spaltungsreaktion entstehenden Polyisocyanate schnell in verdünnter Form als gasförmige Produkte aus der Reaktionszone entfernt werden und damit einer nur geringen thermischen Belastung ausgesetzt werden. Darüber hinaus erfolgt die Abtrennung von durch Rekombination mit gleichzeitig abdestilliertem Alkohol gebildeten Isocyanatourethanen unter schonenden Bedingungen durch Extraktion. Dies hat zur Folge, daß die an sich bekannten Folgereaktionen von Isocyanaten, die unter Umständen von den eingesetzten Carbaminsäureestern katalysiert werden, weitgehend unterdrückt werden und somit ein wesentlich größerer Teil des bei der Spaltungsreaktion gebildeten Polyisocyanats unzersetzt erhalten bleibt und in reiner Form isoliert werden kann.

Die nachfolgenden Beispiele dienen der weiteren Erläuterung des erfindungsgemäßen Verfahrens. Alle Prozentangaben beziehen sich, falls nicht anderslautend vermerkt, auf Molprozente.

Die Ausbeute nach einem ersten Zyklus bedeutet die tatsächliche Ausbeute, die in den Beispielen erhalten wird. Die Ausbeute für kontinuierliche Fahrweise bezieht sich auf die Gesamtmenge an Diisocyanat, die dadurch erhalten wird, daß (1) zusätzliches Diisocyanat aus dem ersten Zyklus gewonnen wird und (2) nicht reagiertes und teilweise reagiertes Carbaminsäureester in nachfolgenden Zersetzungsschriften zu Produkt umgewandelt werden.

Die Ausbeute berechnet sich als Grenzwert einer geometrischen Reihe wie folgt:

$$Y = \frac{Y_1}{1-(S_1-Y_1)/100}$$

mit

Y = Ausbeute für kontinuierliche Fahrweise (%)

$Y_1$ = Ausbeute nach einem ersten Zyklus (%)

$S_1$ = Summe der Gesamtmenge an Diisocyanat sowie nicht reagierten und teilweise reagiertem Carbaminsäurester (%)

Wenn nicht anders angegeben, dient als Spaltungsreaktor ein zylinderförmiger Dünnschichtverdampfer (effektive Länge 300 mm; Durchmesser 35 mm), der mit einem aus Metall gearbeiteten Flügelblattrührer ausgestattet ist, wobei dessen bewegliche Flügel bis an die Wandung des Dünnschichtverdampfers reichen. Zur Einspeisung des zu spaltenden Carbaminsäureesters dient ein am Kopf des Dünnschichtverdampfers angebrachter beheizbarer Tropftrichter. Die Ausschleusung nicht verdampfbarer Reaktionsprodukte erfolgt über einen am Fuß des Dünnschichtverdampfers angebrachten Absperrhahn, die Entnahme der verdampfbaren Komponenten des Reaktionsgemischs erfolgt am Kopf des Dünnschichtverdampfers über einen beheizten Querstromkühler, auf dessen oberem Ende zwei hintereinandergeschaltete Schlangenkühler mit je einem Entnahmeboden für die jeweils anfallenden Kondensate aufgesetzt sind. Die Evakuierung der Spaltungsapparatur erfolgt über eine hinter den Schlangenkühlern angebrachte Kühlfalle mittels einer Drehschieberpumpe.

Das während der Spaltungsreaktion als Fraktion II anfallende isocyanathaltige Kondensat wird, gegebenenfalls nach Zugabe von weiterem Lösungsmittel auf eine für die Extraktion geeignete Temperatur gebracht und in einem beheizbaren Kolben mit Bodenablaß, der mit einem mit Glas umhüllten Blattrührer, einem Thermometer und einem Rückflußkühler ausgerüstet ist, extrahiert. Dazu wird es mit dem Extraktionsmittel vermischt und 30 Minuten innig gerührt. Nach 10 minütigem Stehenlassen lassen sich die beiden gebildeten Phasen trennen, wobei die spezifisch schwerere Phase in manchen Beispielen einer oder mehreren weiteren Extraktionen mit frischem Extraktionsmittel unterworfen wird. Die spezifisch leichteren Phasen werden vereint und, ebenso wie die nach den Extraktionen übrigbleibende schwerere Phase, mittels HPLC auf ihre Zusammensetzung geprüft.

Beispiel 1

Innerhalb von 5 Stunden wird über den auf 100°C thermostatisierten Tropftrichter eine Lösung von 285 g 4,4'-Bis(ethoxycarbonylamino)-diphenylmethan (MDU) und 0,72 g Dibutylzinndilaurat in 300 g Sulfolan (Tropfgeschwindigkeit: 120 g/h) in einen mit 270°C heißem Wärmeträgeröl erhitzten Dünnschichtverdampfer eingespeist. Der über Kopf angebrachte Querstromkühler wird auf 200°C, die beiden Schlangenkühler auf +50°C bzw. -20°C thermostatisiert. Der Druck in der Apparatur beträgt während der Spaltung 4 mbar. Das über Kopf entweichende Gasgemisch wird an den beiden Schlangenkühlern fraktioniert kondensiert. Es werden 85 g Fraktion I (Zusammensetzung: 74 Gew.-% Ethanol; 26 Gew.-% Sulfolan) und 500 g Fraktion II (Zusammensetzung: 55,1 Gew.-% Sulfolan, 28,1 Gew.-% 4,4'-Diisocyanatodiphenylmethan (MDI), 13,5 Gew.-% 4-Ethoxycarbonylamino-4'-isocyanatodiphenylmethan(MIU) und 3,3 Gew.% 4,4'-Bis(ethoxycarbonylamino)diphenylmethan (MDU) aufgefangen.

Anmerkung:

In den nachstehend benutzten Abkürzungen bedeuten "DI" urethanfreie Polyisocyanate, insbesondere Diisocyanat, "IU" teilgespaltenes, Urethan- und Isocyanatgruppen aufweisendes Produkt, insbesondere Isocyanatourethan, und "DU" unverändertes Ausgangsmaterial, insbesondere Diurethan.

Fraktion II wird bei Raumtemperatur viermal mit je 500 g Cyclohexan extrahiert. Laut HPLC enthält die Sulfolanphase nach der letzten Extraktion 45,8 g MDI, 55,2 g MIU sowie 14,5 g MDU, die gesammelten Extrakte enthalten insgesamt 79,8 g MDI und 4,5 g MIU (der MDU-Gehalt liegt unterhalb der Nachweisgrenze). Daraus errechnet sich hinsichtlich der Bildung und Isolierung von 4,4'-Diisocyanatodiphenylmethan (MDI) eine Ausbeute von 38,7 % nach einem ersten Zyklus und eine Ausbeute von 80,3 % für kontinuierliche Fahrweise

Beispiel 2

Gemäß Beispiel 1 wird innerhalb von 6 Stunden eine Lösung von 290 g 4,4'-Bis-(ethoxycarbonylamino)diphenylmethan (MDU) und 0,76 g Dibutylzinndilaurat in 300 g Sulfolan (Tropfgeschwindigkeit: 100 g/h) in einem mit 270°C heißem Wärmeträgeröl erhitzten Dünnschichtverdampfer thermolysiert. Es werden 66 g Fraktion I (Zusammensetzung: 94 Gew.-% Ethanol; 6 Gew.-% Sulfolan) und 510 g Fraktion II (Zusammensetzung: 59,9 Gew.-% Sulfolan; 25,2 Gew.-% MDI; 11,8 Gew.-% MIU und 3,1 Gew.-% MDU) aufgefangen. Nach viermaliger Extraktion der Fraktion II mit je 500 g Cyclohexan bei einer Temperatur von 50°C werden in der Sulfolanphase 24,0 g MDI, 46,2 g MIU sowie 16,1 g MDU gefunden. Die gesammelten Extrakte enthalten insgesamt 114,8 g MDI und 11,2 g MIU (der MDU-Gehalt liegt unterhalb der Nachweisgrenze). Daraus errechnet sich hinsichtlich der Bildung und Isolierung von 4,4'-Diisocyanatodiphenylmethan (MDI) eine Ausbeute von 54.1 % nach einem ersten Zyklus und eine Ausbeute von 90,0 % für kontinuierliche Fahrweise.

Beispiel 3

Gemäß Beispiel 1 wird innerhalb von 5,5 Stunden eine Lösung von 255 g 2,4-Bis-(ethoxycarbonylamino)toluol (TDU) und 0,69 g Dibutylzinndilaurat in 75 g Sulfolan (Tropfgeschwindigkeit: 65 g/h) in einem mit 270°C heißem Wärmeträgeröl erhitzten Dünnschichtverdampfer thermolysiert. Der über Kopf angebrachte Querstromkühler ist dabei auf 150°C, die beiden Schlangenkühler auf +30°C bzw -10°C thermostatisiert. Der Druck in der Apparatur beträgt während der Spaltung 10 mbar. Es werden 67 g Fraktion I (Zusammensetzung: 96 Gew.-% Ethanol; 4 Gew.-% Sulfolan) und 255 g Fraktion II (Zusammensetzung: 34,9 Gew.-% Sulfolan; 23,2 Gew.-% TDI; 35,3 Gew.-% TIU (Isomerengemisch) und 6,7 Gew.-% TDU) aufgefangen. Fraktion II wird nach Zugabe von 350 g Sulfolan bei Raumtemperatur viermal mit je 200 g einer tert.-Butylmethylether/Isooctan-Mischung (1:2) extrahiert. Die gesammelten Extrakte enthalten insgesamt 40,4 g TDI sowie 20,2 g TIU (Isomerengemisch) (der TDU-Gehalt liegt unterhalb der Nachweisgrenze). In der Sulfolanphase werden nach beendeter Extraktion 29,3 g TDI, 72,4 g TIU (Isomerengemisch) und 17,3 g TDU nachgewiesen. Daraus errechnet sich hinsichtlich der Bildung und Isolierung von 2,4-Diisocyanatotoluol (TDI) eine Ausbeute von 24,3 % nach einem ersten Zyklus und eine Ausbeute von 76,7 % für kontinuierliche Fahrweise.

Beispiel 4

Gemäß Beispiel 1 wird innerhalb von 5 Stunden eine Mischung aus 275 g 1-(Ethoxycarbonylamino)-3,3,5-trimethyl-5-(ethoxycarbonylaminomethyl)-cyclohexan (IPDU), 0,70 g Dibutylzinndilaurat und 15 g Sulfolan über den auf 150°C thermostatisierten Tropftrichter (Tropfgeschwindigkeit: 60 g/h) in den mit 310°C heißem Wärmeträgeröl erhitzten Dünnschichtverdampfer eingespeist. Der über Kopf angebrachte Querstromkühler ist dabei auf 150°C, die beiden Schlangenkühler auf +20°C bzw -10°C thermostatisiert. Der Druck in der Apparatur beträgt während der Spaltung 12 mbar. Es werden 63 g Fraktion I (Zusammensetzung: 94 Gew.-% Ethanol; 6 Gew.-% Sulfolan) und 193 g Fraktion II (Zusammensetzung: 3,5 Gew.-% Sulfolan; 45,9 Gew.-% IPDI; 47,0 Gew.-% IPIU (Isomerengemisch)) aufgefangen. (Der bei der Spaltungsreaktion unverändert gebliebene Ausgangscarbaminsäureester (IPDU) wird in der HPLC-Analyse nicht erfaßt). Nach Verdünnen mit 180 g Sulfolan wird Fraktion II bei einer Temperatur von 90°C viermal mit je 360 g Isooctan extrahiert. In der Sulfolanphase werden nach beendeter Extraktion 3,5 g IPDI sowie 15,6 g IPIU (Isomerengemisch) nachgewiesen. Die gesammelten Extrakte enthalten insgesamt 82,5 g IPDI sowie 63,3 g IPIU (Isomerengemisch). Daraus errechnet sich hinsichtlich der Bildung und Isolierung von 1-Isocyanato-3,3,5-trimethyl-5-(isocyanatomethyl)-cyclohexan (IPDI) eine Ausbeute von 42,4 % nach einem ersten Zyklus und eine Ausbeute von 71,6 % für kontinuierliche Fahrweise.

Beispiel 5

Gemäß Beispiel 1 wird innerhalb von 4,5 Stunden eine Lösung von 240 g 1,6-Bis-(ethoxycarbonylamino)hexan (HDU) und 0,66 g Dibutylzinndilaurat in 85 g Sulfolan (Tropfgeschwindigkeit: 70 g/h) in einem mit 270°C heißem Wärmeträgeröl erhitzten Dünnschichtverdampfer thermolysiert. Der über Kopf angebrachte Querstromkühler ist dabei auf 150°C, die beiden Schlangenkühler auf +20°C bzw. -10°C thermostatisiert. Der Druck in der Apparatur beträgt während der Spaltung 15 mbar. Es werden 75 g Fraktion I (Zusammensetzung: 75 Gew.-% Ethanol; 25 Gew.-% Sulfolan) und 245 g Fraktion II (Zusammensetzung: 27,9 Gew.-% Sulfolan; 35,8 Gew.-% HDI; 36,6 Gew.-% HIU) aufgefangen. (Der bei der Spaltungsreaktion unverändert gebliebende Ausgangscarbaminsäureester (HDU) wird in der HPLC-Analytik nicht erfaßt). Fraktion II wird bei einer Temperatur von 90°C viermal mit je 160 g Isooctan extrahiert. Die gesammelten Extrakte enthalten insgesamt 48,6 g HDI sowie 27,6 g HIU. In der Sulfolanphase werden nach beendeter Extraktion 21,8 g HDI sowie 45,1 g HIU nachgewiesen. Daraus errechnet sich hinsichtlich der Bildung und Isolierung von 1,6-Diisocyanatohexan (HDI) eine Ausbeute von 31,4 % nach einem ersten Zyklus und eine Ausbeute von 72,5 % bei kontinuierlicher Fahrweise.

**Patentansprüche**

1. Verfahren zur Herstellung von Polyisocyanaten durch thermische Spaltung der den Polyisocyanaten entsprechenden N-substituierten Carbaminsäureester in Lösung in Gegenwart von bis zu 75 Gew.-% eines Lösungsmittels oder Lösungsmittelgemisches bei oberhalb 150°C liegenden Temperaturen, dadurch gekennzeichnet, daß man

A) den Carbaminsäureester oder das Gemisch von Carbaminsäureestern der Formel

$$R^1(NHCOOR^2)_n$$

in welcher

R$^1$ für einen gegebenenfalls inerte Substituenten aufweisenden aliphatischen Kohlenwasserstoffrest mit insgesamt 4 bis 18 Kohlenstoffatomen, einen gegebenenfalls inerte Substituenten aufweisenden cycloaliphatischen Kohlenwasserstoffrest mit insgesamt 6 bis 25 Kohlenstoffatomen, einen gegebenenfalls inerte Substituenten aufweisenden araliphatischen Kohlenwasserstoffrest mit insgesamt 7 bis 25 Kohlenstoffatomen oder einen gegebenenfalls inerte Substituenten aufweisenden aromatischen Kohlenwasserstoffrest mit insgesamt 6 bis 30 Kohlenstoffatomen steht,

R$^2$ für einen aliphatischen Kohlenwasserstoffrest mit 1 bis 18 Kohlenstoffatomen, einen cycloaliphatischen Kohlenwasserstoffrest mit 5 bis 15 Kohlenstoffatomen, einen araliphatischen Kohlenwasserstoffrest mit 7 bis 10 Kohlenstoffatomen oder einen aromatischen Kohlenwasserstoffrest mit 6 bis 10 Kohlenstoffatomen steht, und

n für eine ganze Zahl von 2 bis 5 steht,

mit der Maßgabe, daß die dem Rest R$^2$ entsprechenden Alkohole R$^2$-OH bei Normaldruck einen mindestens um 10°C unterhalb des Siedepunktes des eingesetzten Lösungsmittels und des dem Rest R$^1$ entsprechenden Polyisocyanats R$^1$(NCO)$_n$ liegenden Siedepunkt aufweisen, als mindestens 25 gew.-%ige Lösung in einem polaren Lösungsmittel oder Lösungsmittelgemisch löst, das

a) eine Dielektrizitätskonstante bei 25°C oberhalb 20 aufweist,

b) die Carbaminsäureester zu lösen vermag,

c) bei der Spalttemperatur stabil und gegenüber den Carbaminsäureestern und den gebildeten Isocyanaten inert ist,

d) einen Siedepunkt mindestens 10°C oberhalb des Siedepunkts des dem zu spaltenden Carbaminsäureester zugrunde liegenden Alkohols aufweist,

e) unter den Spaltbedingungen unzersetzt destillierbar ist,

f) mit dem gemäß D) eingesetzten Extraktionsmittel mindestens eine Mischungslücke aufweist, und

B) die thermische Spaltung in einem Rohrreaktor so durchführt, daß Gemische aus Polyisocyanaten, Isocyanatourethanen und nicht umgesetzten Carbaminsäureestern erhalten werden, deren Isocyanatgehalt mehr als 50 % der Theorie entspricht, wobei die gemäß A) hergestellten Lösungen an der Innenwand des Rohrreaktors entlang geführt werden,

C) die unter den Reaktionsbedingungen anfallenden Gase über Kopf abnimmt und durch fraktionierte Kondensation in eine vorwiegend aus Alkohol bestehende Fraktion I und eine vorwiegend aus Polyisocyanat, Isocyanatourethan, Carbaminsäureester und dem gemäß A) eingesetzten Lösungsmittel bestehende Fraktion II auftrennt,

D) das in Fraktion II enthaltene Polyisocyanat durch Extraktion mit einem Extraktionsmittel mit einem bei 1013 mbar bei 30° C bis 200° C liegenden Siedepunkt bzw. -bereich, welches mit der Fraktion II nicht unbegrenzt mischbar ist und für das Polyisocyanat ein Lösungsmittel darstellt und welches aus der Gruppe der aliphatischen, cycloaliphatischen oder araliphatischen Kohlenwasserstoffe, aliphatischen Ether oder beliebiger Gemische derartiger Lösungsmittel ausgewählt ist, aus Fraktion II entfernt und gegebenenfalls anschließend die dabei anfallende Lösung des Polyisocyanats in dem Extraktionsmittel destillativ aufarbeitet, und

E) den nicht extrahierten Teil des Reaktionsgemisches zurückführt.

2. Verfahren gemäß Anspruch 1, dadurch gekennzeichnet, daß der nicht extrahierte Teil des Reaktionsgemisches in die Spaltungsreaktion gemäß B) zurückgeführt wird.

3. Verfahren gemäß Anspruch 1 und 2, dadurch gekennzeichnet, daß als Lösungsmittel Sulfolan oder 3-Methyl-sulfolan eingesetzt wird.

4. Verfahren gemäß Anspruch 1 bis 3, dadurch gekennzeichnet, daß als Extraktionsmittel Isooctan, Cyclohexan, Toluol und/oder tert.-Butylmethylether eingesetzt werden.

## Claims

1. A process for the production of polyisocyanates by thermal cleavage of the N-substituted carbamic acid esters corresponding to the polyisocyanates in solution in the presence of up to 75% by weight of a solvent or solvent mixture at temperatures above 150°C, characterized in that

A) the carbamic acid ester or the mixture of carbamic acid esters correspond to the formula:

$$R^1(NHCOOR^2)_n$$

in which

$R^1$ is an aliphatic hydrocarbon radical with a total of 4 to 18 carbon atoms optionally bearing inert substituents, a cycloaliphatic hydrocarbon radical with a total of 6 to 25 carbon atoms optionally bearing inert substituents, an araliphatic hydrocarbon radical with a total of 7 to 25 carbon atoms optionally bearing inert substituents or an aromatic hydrocarbon radical with a total of 6 to 30 carbon atoms optionally bearing inert substituents,

$R^2$ is an aliphatic hydrocarbon radical containing 1 to 18 carbon atoms, a cycloaliphatic hydrocarbon radical containing 5 to 15 carbon atoms, an araliphatic hydrocarbon radical containing 7 to 10 carbon atoms or an aromatic hydrocarbon radical containing 6 to 10 carbon atoms and

n is an integer of 2 to 5,

with the proviso that the alcohols $R^2$-OH corresponding to the radical $R^2$ have a boiling point at normal pressure at least 10°C below the boiling point of the solvent used and the polyisocyanate $R^1(NCO)_n$ corresponding to the radical $R^1$, is dissolved to form an at least 25% by weight solution in a polar solvent or solvent mixture which

a) has a dielectric constant above 20 at 25°C,

b) is capable of dissolving the carbamic acid ester,

c) is stable at the cleavage temperature and inert to the carbamic acid esters and to the isocyanates formed,

d) has a boiling point at least 10°C above the boiling point of the alcohol on which the carbamic acid ester to be subjected to cleavage is based,

e) can be distilled without decomposing under the cleavage conditions,

f) has at least one miscibility gap with the extractant used in step D) and

B) the thermal cleavage is carried out in a tube reactor in such a way that mixtures of polyisocyanates, isocyanatourethanes and unreacted carbamic acid esters of which the isocyanate content corresponds to more than 50% of the theoretical are obtained, the solutions prepared in step A) being guided along the inner wall of the tube reactor,

C) the gases accumulating under the reaction conditions being removed overhead and being separated by fractional condensation into a fraction I consisting predominantly of alcohol and a fraction II consisting predominantly of polyisocyanate, isocyanatourethane, carbamic acid ester and the solvent used in step A),

D) the polyisocyanate obtained in fraction II is removed from fraction II by extraction with an extractant which has a boiling point or boiling range of 30°C to 200°C at 1013 mbar, is not indefinitely miscible with fraction II, is a solvent for the polyisocyanate and is selected from the group consisting of aliphatic, cycloaliphatic or araliphatic hydrocarbons, aliphatic ethers or mixtures of such solvents and the solution of the polyisocyanate in the extractant is then optionally worked up by distillation and

E) the non-extracted part of the reaction mixture is returned.

2. A process as claimed in claim 1, characterized in that the non-extracted part of the reaction mixture is returned to the cleavage reaction in step B).

3. A process as claimed in claims 1 and 2, characterized in that sulfolan or 3-methyl sulfolan is used as the solvent.

4. A process as claimed in claims 1 to 3, characterized in that isooctane, cyclohexane, toluene and/or tert.butyl methyl ether is/are used as the extractant.

**Revendications**

1. Procédé de préparation de polyisocyanates par scission à la chaleur des esters carbamiques N-substitués correspondant aux polyisocyanates, en solution, en présence d'une proportion allant jusqu'à 75 % en poids d'un solvant ou mélange solvant, à des températures supérieures à 150°C, caractérisé en ce que

A) on dissout l'ester carbamique ou le mélange d'esters carbamiques de formule :

$R^1(NHCOOR^2)_n$

dans laquelle

$R^1$ représente un radical hydrocarboné aliphatique portant éventuellement des substituants inertes et contenant au total 4 à 18 atomes de carbone, un radical hydrocarboné cycloaliphatique portant éventuellement des substituants inertes et contenant au total 6 à 25 atomes de carbone, un radical hydrocarboné araliphatique portant éventuellement des substituants inertes et contenant au total 7 à 25 atomes de carbone ou un radical hydrocarboné aromatique portant éventuellement des substituants inertes et contenant au total 6 à 30 atomes de carbone,

$R^2$ représente un radical hydrocarboné aliphatique en $C_1$-$C_{18}$, un radical hydrocarboné cycloaliphatique en $C_5$-$C_{15}$, un radical hydrocarboné araliphatique en $C_7$-$C_{10}$ ou un radical hydrocarboné aromatique en $C_6$-$C_{10}$ et

n est un nombre entier allant de 2 à 5,

sous réserve que les alcools $R^2$-OH correspondant au radical $R^2$ ont, à pression normale, un point d'ébullition situé à au moins 10°C au-dessous du point d'ébullition du solvant mis en oeuvre et du point d'ébullition du polyisocyanate $R^1(NCO)n$ correspondant au radical $R^1$, à l'état de solution à une concentration d'au moins 25 % en poids, dans un solvant ou mélange solvant polaire qui

a) a une constante diélectrique supérieure à 20 à 25°C,

b) est capable de dissoudre l'ester carbamique,

c) est stable à la température de scission et inerte à l'égard des esters carbamiques et des isocyanates formés,

d) a un point d'ébullition situé à au moins 10°C au-dessus du point d'ébullition de l'alcool dont dérive l'ester carbamique soumis à la scission,

e) peut être distillé sans décomposition dans les conditions de la scission,

f) a au moins un intervalle de non-miscibilité avec l'argent d'extraction utilisé en D), et

B) on procède à la scission thermique dans un réacteur tubulaire de manière à obtenir des mélanges de polyisocyanate, d'isocyanatouréthanne et d'ester carbamique non converti dont la teneur en isocyanate est supérieure à 50 % de la théorie, en envoyant les solutions préparées en A) sur la paroi intérieure du réacteur tubulaire,

**C)** on évacue en tête les gaz formés dans les conditions de la réaction et on les sépare par condensation fractionnée en une fraction I consistant principalement en l'alcool et une fraction II consistant principalement en le polyisocyanate, l'isocyanatouréthanne, l'ester carbamique et le solvant utilisé en **A**),

**D)** on sépare de la fraction II le polyisocyanate qu'elle contient par extraction à l'aide d'un agent d'extraction ayant un point ou intervalle d'ébullition entre 30 et 200°C à 1 013 mbar, qui n'est pas miscible en toutes proportions avec la fraction II et est solvant du polyisocyanate et qui est choisi dans le groupe formé par les hydrocarbures aliphatiques, cycloaliphatiques ou araliphatiques, les esters aliphatiques et les mélanges quelconques de ces solvants et, le cas échéant, on traite la solution du polyisocyanate dans l'agent d'extraction par distillation, et

**E)** on recycle la partie non extraite du mélange de réaction.

2. Procédé selon revendication 1, caractérisé en ce que la partie non extraite du mélange de réaction est recyclée à la réaction de scission **B**).

3. Procédé selon les revendications 1 et 2, caractérisé en ce que l'on utilise en tant que solvant le sulfolan ou le 3-méthylsulfolan.

4. Procédé selon les revendications 1 à 3, caractérisé en ce que l'on utilise en tant qu'agents d'extraction, l'isooctane, le cyclohexane, le toluène et/ou l'éther tert-butylméthylique.